(19) Europäisches Patentamt
European Patent Office
Office européen des brevets

(11) **EP 4 265 104 A1**

(12) **EUROPEAN PATENT APPLICATION**
published in accordance with Art. 153(4) EPC

(43) Date of publication:
**25.10.2023 Bulletin 2023/43**

(21) Application number: **22739380.8**

(22) Date of filing: **11.01.2022**

(51) International Patent Classification (IPC):
**A01H 1/00** (2006.01)     **A01H 3/02** (2006.01)
**A01G 7/00** (2006.01)     **A01H 6/82** (2018.01)

(52) Cooperative Patent Classification (CPC):
**A01G 7/00; A01H 1/00; A01H 3/02; A01H 6/82**

(86) International application number:
**PCT/JP2022/000548**

(87) International publication number:
**WO 2022/153973 (21.07.2022 Gazette 2022/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.01.2021 JP 2021004819**

(71) Applicant: **Denka Company Limited
Tokyo 103-8338 (JP)**

(72) Inventors:
• **WAKABAYASHI Yuki
Tokyo 103-8338 (JP)**
• **KAKEMA Teruaki
Tokyo 103-8338 (JP)**

(74) Representative: **Hoffmann Eitle
Patent- und Rechtsanwälte PartmbB
Arabellastraße 30
81925 München (DE)**

(54) **METHOD FOR DETERMINING CONDITIONS FOR CULTIVATION, AND METHOD FOR PRODUCING DESIRED PROTEIN OR DESIRED PEPTIDE**

(57) Disclosed is a method for determining conditions for cultivating a plant body expressing a protein of interest or a peptide of interest in a 24-hour cycle in which a dark period and a light period are repeated alternately, including: a step of determining an optimum leaf temperature based on a capability to express a protein of interest or a peptide of interest; and a step of setting light and dark period leaf temperatures and light and dark period time lengths after a plant body is infected with a bacterium or a virus having a polynucleotide encoding the protein of interest or the peptide of interest so as to satisfy the following formula, the following formula being: Dark period leaf temperature (°C) $\times$ Dark period time length (hour) + Light period leaf temperature (°C) $\times$ Light period time length (hour) = Optimum leaf temperature (°C) $\times$ 24 (hour) $\pm$ 24.

**Description**

**Technical Field**

The present invention relates to a method for determining conditions for cultivation and a method for producing a protein of interest or a peptide of interest.

**Background Art**

Various methods involving heterologously expressing a protein of interest or a peptide of interest in plant bodies by gene recombination techniques and the like to produce the protein of interest or the peptide of interest have been developed. For example, plant bodies are often infected with bacteria or viruses having polynucleotides encoding proteins of interest or peptides of interest to heterologously express the proteins of interest or the peptides of interest in the plant bodies (Non Patent Literature 1). Although there are many reports on methods for heterologously expressing proteins of interest or peptides of interest in plant bodies and then collecting the proteins or interest or the peptides or interest, the conditions for cultivating plant bodies after the plant bodies were infected with bacteria or viruses for heterologous expression have hardly been examined.

**Citation List**

**Non Patent Literature**

Non Patent Literature 1: Marillonnet et al. "In planta engineering of viral RNA replicons: efficient assembly by recombination of DNA modules delivered by Agrobacterium. ", Proc Natl Acad Sci US A., 2004 Oct 26;101(43): 15546

**Summary of Invention**

**Technical Problem**

An object of the present invention is to provide a new method for determining conditions for cultivating a plant body when a protein of interest or a peptide of interest is expressed in the plant body and a method for producing the protein of interest or the peptide of interest by cultivating the plant body under the determined conditions for cultivation.

**Solution to Problem**

The present inventors have found that suitable conditions for cultivation can be determined based on the leaf temperatures during the cultivation and completed the present invention.
That is, the present invention includes, for example, the following inventions.

[1] A method for determining conditions for cultivating a plant body expressing a protein of interest or a peptide of interest in a 24-hour cycle in which a dark period and a light period are repeated alternately, including:

a step of determining an optimum leaf temperature based on a capability to express a protein of interest or a peptide of interest and
a step of setting light and dark period leaf temperatures and light and dark period time lengths after a plant body is infected with a bacterium or a virus having a polynucleotide encoding the protein of interest or the peptide of interest so as to satisfy the following formula, , the following formula being:

$$\text{Dark period leaf temperature (℃)} \times \text{Dark period time length (hour)} + \text{Light period leaf temperature (℃)} \times \text{Light period time length (hour)} = \text{Optimum leaf temperature (℃)} \times 24 \text{ (hour)} \pm 24.$$

[2] The method according to [1], wherein the step of determining the optimum leaf temperature includes determining the optimum leaf temperature based on an expression level of the protein of interest or the peptide of interest.
[3] The method according to [1] or [2], including:

setting the leaf temperature in the light period higher than the leaf temperature in the dark period.

[4] The method according to any one of [1] to [3], wherein as a breakdown of the 24-hour cycle, the dark period is set as a time length between 3 hours and 21 hours, the light period is set as a time length between 3 hours and 21 hours, and a total time length of the dark period and the light period is 24 hours.

[5] The method according to any one of [1] to [4], wherein the optimum leaf temperature is a temperature between 22°C and 27°C.

[6] The method according to any one of [1] to [5], wherein the dark period leaf temperature is a temperature between 16°C and 27°C, and the light period leaf temperature is a temperature between 16°C and 27°C.

[7] The method according to any one of [1] to [6], wherein the plant body is a *Nicotiana* plant.

[8] The method according to any one of [1] to [7], wherein the protein of interest or the peptide of interest is an antibody, an enzyme, a hormone, or an antigen.

[9] A method for producing a protein of interest or a peptide of interest, including:

determining conditions for cultivation by the method according to any one of [1] to [8];
cultivating a plant body expressing a protein of interest or a peptide of interest under the determined conditions for cultivation; and
extracting the protein of interest or the peptide of interest from the cultivated plant body.

[10] A method for determining conditions for cultivating a plant body expressing a protein of interest or a peptide of interest in a cycle in which a dark period and a light period are repeated alternately, including:

a step of determining an optimum leaf temperature based on a capability to express a protein of interest or a peptide of interest and
a step of setting light and dark period leaf temperatures and light and dark period time lengths after a plant body is infected with a bacterium or a virus having a polynucleotide encoding the protein of interest or the peptide of interest so as to satisfy the following formula, , the following formula being: Dark period leaf temperature (°C) × Dark period time length per cycle (hour) + Light period leaf temperature (°C) × Light period time length per cycle (hour) = Optimum leaf temperature (°C) × Total time length of 1 cycle ± 24.

[11] The method according to [10], wherein the step of determining the optimum leaf temperature includes determining the optimum leaf temperature based on an expression level of the protein of interest or the peptide of interest.

[12] The method according to [10] or [11], including:
setting the leaf temperature in the light period higher than the leaf temperature in the dark period.

[13] The method according to any one of [10] to [12], wherein the dark period is set as a time length between 3 hours and 21 hours, and the light period is set as a time length between 3 hours and 21 hours.

[14] The method according to any one of [10] to [13], wherein the optimum leaf temperature is a temperature between 22°C and 27°C.

[15] The method according to any one of [10] and [14], wherein the dark period leaf temperature is a temperature between 16°C and 27°C, and the light period leaf temperature is a temperature between 16°C and 27°C.

[16] The method according to any one of [10] to [15], wherein the plant body is a *Nicotiana* plant.

[17] The method according to any one of [10] to [16], wherein the protein of interest or the peptide of interest is an antibody, an enzyme, a hormone, or an antigen.

[18] The method according to any one of [10] to [17], wherein the 1 cycle is set as a time length between 20 hours and 36 hours.

[19] A method for determining conditions for cultivating a plant body expressing a protein of interest or a peptide of interest in a 24-hour cycle including a plurality of temperature zones, including:

a step of determining an optimum leaf temperature based on a capability to express a protein of interest or a peptide of interest and
a step of setting a leaf temperature and a number of measurements so that an integrated value of leaf temperatures measured at regular intervals after a plant body is infected with a bacterium or a virus having a polynucleotide encoding the protein of interest or the peptide of interest satisfies the following relationship:

$$(T - 1) \times (C_1 + \cdots + C_n) \leq (T_1 \times C_1) + \cdots + (T_n \times C_n) \leq (T + 1) \times (C_1 + \cdots + C_n)$$

$$n \times \text{measurement interval (hour)} = 24 \text{ (hour)}$$

$$C_1 + \cdots + C_n = 24 \text{ (hour)}$$

wherein T is the optimum leaf temperature (°C), $T_1$ to $T_n$ are leaf temperatures (°C), n is the number of measurements (time), and n is an integer of 2 or more and 10 or less.

[20] The method according to [19], wherein n is 2 or 3.

[21] A method for producing a protein of interest or a peptide of interest, including:

determining conditions for cultivation by the method according to [19] or [20];
cultivating a plant body expressing a protein of interest or a peptide of interest under the determined conditions for cultivation; and extracting the protein of interest or the peptide of interest from the cultivated plant body.

[22] A method for determining conditions for cultivating a plant body expressing a protein of interest or a peptide of interest in a fixed time (C (hour)) cycle including a plurality of temperature zones, including:

a step of determining an optimum leaf temperature based on a capability to express a protein of interest or a peptide of interest and
a step of setting a leaf temperature and a number of measurements so that an integrated value of leaf temperatures measured at regular intervals after a plant body is infected with a bacterium or a virus having a polynucleotide encoding the protein of interest or the peptide of interest satisfies the following relationship:

$$(T - 1) \times (C_1 + \cdots + C_n) \leq (T_1 \times C_1) + \cdots + (T_n \times C_n) \leq (T + 1) \times (C_1 + \cdots + C_n)$$

$$n \times \text{measurement interval (hour)} = C \text{ (hour)}$$

$$C_1 + \cdots + C_n = C \text{ (hour)}$$

wherein T is the optimum leaf temperature (°C), $T_1$ to $T_n$ are leaf temperatures (°C), n is the number of measurements (time), and n is an integer of 2 or more and 10 or less.

[23] The method according to [22], wherein n is 2 or 3.

[24] A method for producing a protein of interest or a peptide of interest, including:

determining conditions for cultivation by the method according to [22] or [23];
cultivating a plant body expressing a protein of interest or a peptide of interest under the determined conditions for cultivation; and
extracting the protein of interest or the peptide of interest from the cultivated plant body.

**Advantageous Effects of Invention**

According to the present invention, a new method for determining conditions for cultivating a plant body when a protein of interest or a peptide of interest is expressed in the plant body and a method for producing the protein of interest or the peptide of interest by cultivating the plant body under the determined conditions for cultivation can be provided.

According to the present invention, the light and dark period leaf temperatures and the light and dark period time lengths do not need to be maintained constant; and as long as conditions of the present invention are satisfied, the light and dark period leaf temperatures and the light and dark period times can be flexibly set, and the degree of freedom of the setting of the conditions for cultivation increases. Air conditioning electric power cost during the cultivation in the light period can be expected to be reduced by adjusting the light and dark period leaf temperatures and the light and dark period time lengths, for example, setting the leaf temperature in the light period higher than the leaf temperature in the dark period.

**Brief Description of Drawings**

[Figure 1] Figure 1 is a graph showing the results of performing cultivation after the infection under conditions in Table 1 and comparing the expression levels of immunoglobulin G.
[Figure 2] Figure 2 is a graph showing the results of performing cultivation after the infection under conditions in Table 2 and comparing the expression levels of immunoglobulin G.

**Description of Embodiments**

As an embodiment, the present invention provides a method for determining conditions for cultivating a plant body expressing a protein of interest or a peptide of interest in a 24-hour cycle in which a dark period and a light period are repeated alternately, including:

a step of determining an optimum leaf temperature based on a capability to express a protein of interest or a peptide of interest and
a step of setting light and dark period leaf temperatures and light and dark period time lengths after a plant body is infected with a bacterium or a virus having a polynucleotide encoding the protein of interest or the peptide of interest so as to satisfy the following formula is satisfied, , the following formula being: Dark period leaf temperature (°C) × Dark period time length (hour) + Light period leaf temperature (°C) × Light period time length (hour) = Optimum leaf temperature (°C) × 24 (hour) ± 24.

As an embodiment, the present invention also provides a method for determining conditions for cultivating a plant body expressing a protein of interest or a peptide of interest in a cycle in which a dark period and a light period are repeated alternately, including:

a step of determining an optimum leaf temperature based on a capability to express a protein of interest or a peptide of interest and
a step of setting light and dark period leaf temperatures and light and dark period time lengths after a plant body is infected with a bacterium or a virus having a polynucleotide encoding the protein of interest or the peptide of interest so as to satisfy the following formula, , the following formula being: Dark period leaf temperature (°C) × Dark period time length per cycle (hour) + Light period leaf temperature (°C) × Light period time length per cycle (hour) = Optimum leaf temperature (°C) × Total time length of 1 cycle ± 24.

As long as the protein of interest or the peptide of interest can be expressed in the plant body, the protein of interest or the peptide of interest is not particularly limited, and may be, for example, a protein or a peptide that exists in the nature, a protein or a peptide derived from a protein or a peptide that exists in the nature, or a protein or a peptide designed artificially. Examples of the protein of interest or the peptide of interest include bioactive proteins such as enzymes, antibodies, and hormones; antigens; epitopes; growth factors; cytokines; transcription factors; receptors; and partial peptides thereof. For example, the protein of interest or the peptide of interest may be selected from group consisting of an enzyme, an antigen, a hormone, and an antibody, or may be an enzyme. The derivation of the protein of interest or the peptide of interest is not limited, either, and the protein of interest or the peptide of interest may be derived, for example, from an animal (including a mammal such as a human), a plant, a filamentous fungus, a bacterium, or yeast.

Examples of the enzymes include oxidases, reductases, lipases (for example, phospholipases), proteases, kinases, phosphatases, cellulases, steroid synthases, methylases, demethylases, collagenases, transglutaminases, glycosidases, and chitinases. For example, the enzyme may be selected from the group consisting of a phospholipase, an esterase, and an oxidase, or may be an esterase, or may be a phosphodiesterase.

Examples of the antibody include complete antibodies (for example, IgG, IgM, IgA, IgD, and IgE), Fab, F(ab'), F(ab')2, Fc, Fc fusion proteins, heavy chains (H chains), light chains (L chains), single-chain Fv (scFv), sc(Fv)2, disulfide bond Fv (sdFv), and diabodies.

Example of the antigens and the epitopes include proteins derived from filamentous fungi, proteins derived from bacteria, and proteins derived from viruses. Examples of the antigens includes self-assembled protein multimers such as virus-like particles (VLPs). As long as the antigenic proteins or the epitopes to be used as vaccines have immunogenicity, the antigenic proteins or the epitopes are not particularly limited, and examples of the antigenic proteins or the epitopes include proteins derived from pathogenic filamentous fungi, proteins derived from pathogenic bacteria, and proteins derived from pathogenic viruses.

Examples of the growth factors include epidermal growth factors (EGFs), insulin-like growth factors (IGFs), transforming growth factors (TGFs), fibroblast growth factors (FGFs), nerve growth factors (NGF), brain-derived neurotrophic factors (BDNFs), vascular endothelial cell growth factor (VEGFs), granulocyte colony stimulating factors (G-CSFs), granulocyte macrophage colony stimulating factors (GM-CSFs), platelet-derived growth factors (PDGFs), erythropoietin (EPO), thrombopoietin (TPO), and hepatocyte growth factors (HGFs).

Examples of the hormones include peptide/protein hormones.

Examples of the cytokines include interleukins (ILs), hematopoietic factors (CSF, EPO, and TPO), interferons (IFN-$\alpha$, IFN-$\beta$, and IFN-$\gamma$), tumor necrosis factors (TNFs), growth factors (EGF, FGF, and PDGF), and chemokines (IL-8).

Examples of the transcription factors include general transcription factors, upstream transcription factors, and inductive transcription factors.

Examples of the receptors include G protein-binding receptors, ionotropic receptors, and a cytokine receptor superfamily.

The protein of interest or the peptide of interest may be a fusion protein in which two or more proteins are bound. The fusion protein of two or more proteins may be a fusion protein of two or more heterologous proteins or a fusion protein of two or more homologous proteins.

Although the molecular weight of the protein of interest or the peptide of interest is not particularly limited, for example, the molecular weight may be 1 kDa to 300 kDa, 1 kDa to 200 kDa, 1 kDa to 100 kDa, 1 kDa to 75 kDa, 1 kDa to 50 kDa, 1 kDa to 10 kDa, 20 kDa to 500 kDa, 50 kDa to 300 kDa, or 100 kDa to 200 kDa. For example, the molecular weight of the protein of interest or the peptide of interest may be 10 kDa or more, 20 kDa or more, 50 kDa or more, 80 kDa or more, 100 kDa or more, 500 kDa or less, 300 kDa or less, 200 kDa or less, 100 kDa or less, 75 kDa or less, or 50 kDa or less.

The protein of interest or the peptide of interest may be a monomer, a dimer, a trimer, or a multimer.

A tag for detecting and/or purifying the protein of interest or the peptide of interest easily may be added to the protein of interest or the peptide of interest.

The plant body that expresses the protein of interest or the peptide of interest is produced by infecting a plant body with a bacterium or a virus having a polynucleotide encoding the protein of interest or the peptide of interest. Examples of such methods include agroinfiltration, the plant virus vector method, the Geneware system, and the magnICON(R) system.

The bacterium or the virus having the polynucleotide encoding the protein of interest or the peptide of interest can be produced, for example, by transforming a bacterium or a virus with the polynucleotide encoding the protein of interest or the peptide of interest. Although the transformation can be performed by a well-known method, for example, a vector containing the polynucleotide encoding the protein of interest or the peptide of interest can be used. Examples of the vector include plasmids, viruses, cosmids, $\lambda$ phage, and artificial chromosomes. It is preferable that the vector be an expression vector containing a promoter sequence and the like. The transformation of a bacterium or a virus with the vector having the polynucleotide encoding the protein of interest or the peptide of interest enables producing the bacterium or the virus having the polynucleotide encoding the protein of interest or the peptide of interest.

Examples of the bacterium to be transformed with the polynucleotide encoding the protein of interest or the peptide of interest include bacteria of *Agrobacterium species, Rhizobium species, Sinorhizobium species, Mesorhizobium species, Phyllobacterium species, Ochrobactrum species,* and *Bradyrhizobium species,* and it is preferable that the bacterium belongs to *Agrobacterium.*

Viruses to be used as virus vectors are also included in viruses to be transformed with the polynucleotide encoding the protein of interest or the peptide of interest. Examples of such viruses include tobacco mosaic virus (TMV), plum pox virus (PPV), potato X virus (PVX), alfalfa mosaic virus (AIMV), cucumber mosaic virus (CMV), cowpea mosaic virus (CPMV), and zucchini yellow mosaic virus (ZYMV).

When agroinfiltration is used, the plant body that expresses the protein of interest or the peptide of interest can be obtained by transforming an *Agrobacterium* bacterium with a T-DNA in which the polynucleotide encoding the protein of interest or the peptide of interest is inserted and infecting the plant body with the *Agrobacterium* bacterium.

When the plant virus vector method is used, the plant body that expresses the protein of interest or the peptide of interest can be obtained by inoculating an RNA obtained from the cDNA of the plant virus genome in which the polynucleotide encoding the protein of interest or the peptide of interest is inserted into the plant body as a vector for infection. Examples of such a virus vector include a tobacco mosaic virus (TMV) vector, a plum pox virus (PPV) vector, a potato X viruse (PVX) vector, an alfalfa mosaic virus (AIMV) vector, a cucumber mosaic virus (CMV) vector, a cowpea mosaic virus (CPMV) vector, and a zucchini yellow mosaic virus (ZYMV) vector.

When the magnICON(R) system is used, the plant body that expresses the protein of interest or the peptide of interest can be obtained by introducing the cDNA of the TMV or PVX genome in which the polynucleotide encoding the protein of interest or the peptide of interest is inserted into a T-DNA vector and infecting a plant body with an Agrobacterium bacterium transformed with the obtained T-DNA vector.

Examples of the plant bodies include, but are not particularly limited to, plants belonging to *Solanaceae* (for example, tobacco, eggplants, tomatoes, green peppers, and red peppers), *Rosaceae* (for example, roses and strawberries), *Brassicaceae* (for example, *Arabidopsis thaliana,* rape, Chinese cabbages, cabbages, Japanese radishes, and rapeseeds), *Asteraceae* (for example, chrysanthemums, crown daisies, lettuces), *Chenopodiaceae* (for example, spinach and sugar beets), *Gramineae* (for example, wheat, rice, barley, corn), and *Leguminosae* (for example, soy beans, adzuki beans, kidney beans, and broad beans). For example, the plant body may be a plant of *Solanaceae* or *Brassicaceae,* may be a plant of *Nicotiana* or *Arabidopsis,* or may be *Nicotiana benthamiana, Nicotiana tabacum,* or *Arabidopsis thaliana.*

The method for infecting the plant body with the bacterium or the virus having the polynucleotide encoding the protein of interest or the peptide of interest can be performed by a well-known method. When the bacterium is an *Agrobacterium* bacterium, the infection can be performed, for example, by infiltration (for example, vacuum infiltration). In the case of the virus, the infection can be performed, for example, by mechanical inoculation. The whole plant body may be infected, or only portions of the plant body (for example, leaves) may be infected.

For example, a liquid in which a culture solution containing the *Agrobacterium* bacterium having the polynucleotide encoding the protein of interest or the peptide of interest is diluted with a buffer solution is used as an infiltration buffer, and the infiltration buffer can be infiltrated into the plant body by vacuum infiltration for infection.

The timing of the plant body infection can be appropriately set depending on the type of the plant body, and the like, and when the plant body is a *Nicotiana* plant, for example, the plant body at 4 weeks to 7 weeks of age, 5 weeks to 6 weeks of age, or 5 weeks of age may be infected.

For example, the 1 cycle in the cultivation under the cycle a dark period and a light period are repeated alternately may be set, for example, as 12 hours, a time length between 12 hours and 48 hours, a time length between 16 hours and 40 hours, a time length between 20 hours and 36 hours, a time length between from 24 hours and 30 hours, or 24 hours.

The whole cultivation period may be 35 days to 56 days, 42 days to 49 days, or 42 days after the sowing, and the cultivation period after the infection may be 4 days to 14 days, 6 days to 9 days, or 7 days.

An environment for cultivating the plant body can be determined depending on the type of the plant body, the type of the protein of interest or the peptide of interest, the type of the bacterium or the virus to be used, and the like. The plant body may be set in culture soil, or may be subjected to water culture. However, it is preferable that the cultivation environment be an environment in which the brightness (illuminance) and the temperature can be controlled during the cultivation, and it is more preferable that the cultivation environment be an environment in which the brightness (illuminance), the temperature, and the humidity can be controlled. Examples of such a cultivation environment include a plant rearing cabinet including artificial lighting, and specific examples thereof include a growth chamber (FC-700Z3, ESPEC MIC CORP.).

As long as the plant can be grown, or the protein of interest can be produced, the temperature in the cultivation is not particularly limited, and may be always constant through all the periods, or may be changed according to the growth of the plant, depending on whether it is day time or night time (in the light period or the dark period), or depending on whether it is before or after the infection. For example, the temperature in the cultivation may be 10°C to 35°C, 15°C to 40°C, 13°C to 28°C, or 15°C to 25°C.

As long as the plant can be grown, or the protein of interest can be produced, the relative humidity in the cultivation is not particularly limited, and may be always constant through all the periods, or may be changed according to the growth of the plant, depending on whether it is day time or night time (in the light period or the dark period), or depending on whether it is before or after the infection. For example, the relative humidity in the cultivation may be 45% to 90%, or 60% to 85%.

As long as the plant can be grown, or the protein of interest can be produced, the $CO_2$ concentration in the cultivation is not particularly limited, and may be always constant through all the periods, or may be changed according to the growth of the plant, depending on whether it is day time or night time (in the light period or the dark period), or depending on whether it is before or after the infection. For example, the $CO_2$ concentration in the cultivation may be 300 ppm to 1000 ppm, or 400 ppm to 700 ppm.

As long as the plant can be grown, or the protein of interest can be produced, the photosynthetic photon flux density in the cultivation is not particularly limited, and may be always constant through all the periods, or may be changed according to the growth of the plant or depending on whether it is before or after the infection. For example, the photosynthetic photon flux density in the cultivation may be 30 $\mu$mol•m$^{-2}$•s$^{-1}$ or more, 30 $\mu$mol•m$^{-2}$•s$^{-1}$ to 1000 $\mu$mol•m$^{-2}$•s$^{-1}$, or 200 $\mu$mol•m$^{-2}$•s$^{-1}$ to 500 $\mu$mol•m$^{-2}$•s$^{-1}$.

The method in the present embodiment includes a step of determining the optimum leaf temperature based on the capability to express the protein of interest or the peptide of interest. For example, a leaf temperature at which the capability to express the protein of interest or the peptide of interest is the highest and/or a leaf temperature at which the capability to express the protein of interest or the peptide of interest is the steadiest can be determined as the optimum leaf temperature. The determination of the optimum leaf temperature enables expressing the protein of interest or the peptide of interest efficiently. For example, the capability to express the protein of interest or the peptide of interest can be determined using the expression level of the protein of interest or the peptide of interest, the expression level of the polynucleotide encoding the protein of interest or the peptide of interest, the efficiency in the translation of the protein of interest or the peptide of interest, and the like as indices. For example, a plurality of plant bodies of the same type as the plant body that expresses the protein of interest or the peptide of interest is infected with the bacterium or the virus having the polynucleotide encoding the protein of interest or the peptide of interest and cultivated at temperatures that are different leaf temperatures, respectively, the expression levels of the protein of interest or the peptide of interest or the expression levels of the polynucleotide encoding the protein of interest or the peptide of interest is then measured, and a leaf temperature at which the highest expression level is exhibited can be determined as the optimum leaf temperature.

For example, the expression level of the protein of interest or the peptide of interest can be measured as follows.

(1) A plant body or a portion (for example, a leaf) of a plant body after infection is collected.
(2) The harvest is milled, an extraction buffer solution is added thereto, followed by centrifugation, and the supernatant is then collected to prepare an extract solution.
(3) The expression level of the protein of interest or the peptide of interest is measured by immunoassay using the supernatant.

Examples of the immunoassay include ELISA and Western blotting.

Here, the collected plant body or the collected portion of the plant body after the infection (harvest) is dry-heated, and the dry weight percentage (%) can be then calculated from the following formula.

$$\text{Dry matter weight percentage (\%)} = \text{Weight of harvest after drying}/\text{Weight of harvest before drying} \times 100$$

Furthermore, the expression level of the protein of interest or the peptide of interest is measured, and the protein amount per harvest after the drying (mg/g dry harvest) can be then calculated from the following formula.

$$\text{Protein amount per harvest after drying (mg/g dry harvest)} = \text{Protein concentration in extract solution (measured value)} \times \text{Extract solution amount (defined as twice the milled harvest weight)}/\text{Milled harvest weight} \times \text{Dry matter weight percentage (\%)}/100$$

For example, the expression level of the protein of interest or the peptide of interest may be calculated as the protein amount per harvest after the drying (mg/g dry harvest), or may be calculated as the protein amount per DL (dry leaf) after the drying (mg/g DL). The expression level of the protein of interest or the peptide of interest varies depending on the plant body, the bacterium, the virus, and the like to be used, and when the cDNA of the TMV or PVX genome in which the polynucleotide encoding the protein of interest or the peptide of interest is inserted is introduced into the T-DNA vector, and a *Nicotiana* plant is infected with an *Agrobacterium* bacterium transformed with the obtained T-DNA vector to express the protein of interest or the peptide of interest, for example, the protein amount per DL (dry leaf) after the drying (mg/g DL) may be 8 (mg/g DL) or more, 10 (mg/g DL) or more, 10 (mg/g DL) to 20 (mg/g DL), or 10 (mg/g DL) to 15 (mg/g DL).

The leaf temperature can be measured with contactless thermometric means such as a radiation thermometer or a thermography device or contact thermometric means such as a thermocouple thermometer. Examples of the thermocouple thermometer include a temperature logger, and specific examples include a temperature logger (Ondotori TR-52i, T&D Corporation). If the plant body is a *Nicotiana* plant, for example, the measurement can be performed by stabbing a leaf of the *Nicotiana* plant from the front side thereof with the temperature logger probe and contacting the probe with the leaf on the back side thereof. If the leaf temperatures are measured in a plurality of leaves and/or a plurality of plants, the average value calculated by dividing the total value of the leaf temperatures by the number of the leaves and/or the number of the plant individuals can be used as the leaf temperature (average leaf temperature).

Although the optimum leaf temperature varies depending on the types of the plant body to be used and the protein of interest or the peptide of interest, and the like, for example, the optimum leaf temperature may be a temperature between 15°C and 40°C, a temperature between 18°C and 37°C, a temperature between 20°C and 30°C, a temperature between 22°C and 27°C, a temperature between 22°C and 25°C, a temperature between 23°C and 25°C, or 23°C. If the plant body is a *Nicotiana* plant, for example, the optimum leaf temperature may be a temperature between 22°C and 27°C, a temperature between 22°C and 25°C, a temperature between 23°C and 25°C, or 23°C.

In the method in the present embodiment, the light and dark period leaf temperatures and the light and dark period time lengths after the plant body is infected with the bacterium or the virus having the polynucleotide encoding the protein of interest or the peptide of interest are set so as to satisfy the following formula (condition).

$$\text{Dark period leaf temperature (°C)} \times \text{Dark period time length per cycle}$$
$$\text{(hour)} + \text{Light period leaf temperature (°C)} \times \text{Light period time length}$$
$$\text{per cycle (hour)} = \text{Optimum leaf temperature (°C)} \times \text{Total time length of}$$
$$\text{1 cycle} \pm 24.$$

For example, if the 1 cycle is 24 hours, the light and dark period leaf temperatures and the light and dark period time lengths after the plant body is infected with the bacterium or the virus having the polynucleotide encoding the protein of interest or the peptide of interest are set so as to satisfy the following formula.

$$\text{Dark period leaf temperature (°C)} \times \text{Dark period time length (hour)} +$$
$$\text{Light period leaf temperature (°C)} \times \text{Light period time length (hour)} =$$
$$\text{Optimum leaf temperature (°C)} \times 24 \text{ (hour)} \pm 24$$

For example, "± 24" in the formula can be changed into "± 20", "± 17", "± 15", "± 10", "± 8", or "± 5".

The temperature for cultivating the plant body after the infection can be determined so as to be light and dark period leaf temperatures set so as to satisfy the above-mentioned condition. The temperatures for cultivating the plant may be constant or changed during a continuous dark period and during a continuous light period.

As long as the above-mentioned condition is satisfied, for example, the leaf temperature in the light period can be set higher than the leaf temperature in the dark period by adjusting the light and dark period leaf temperatures and the light and dark period time lengths. The air conditioning electric power cost during the cultivation in the light period is expected to be reduced by setting the leaf temperature in the light period higher than the leaf temperature in the dark period.

For example, the dark period leaf temperature may be a temperature between 10°C and 35°C, a temperature between 13°C and 28°C, or a temperature between 15°C and 25°C. For example, the light period leaf temperature may be a temperature between 10°C and 35°C, a temperature between 13°C and 28°C, or a temperature between 15°C and 25°C. For example, the dark period leaf temperature is a temperature between 16°C and 27°C, and the light period leaf temperature may be a temperature between 16°C and 27°C; the dark period leaf temperature is a temperature between 16°C and 27°C, and the light period leaf temperature may be a temperature between 20°C and 27°C; and the dark period leaf temperature is a temperature between 16°C and 26°C, and the light period leaf temperature may be a temperature between 22°C and 27°C. It is preferable that the dark period leaf temperature and the light period leaf temperature are set as different temperatures from the viewpoint of exhibiting the effect of the present invention remarkably.

For example, the 1 cycle (total time length of the dark period and the light period) may be set as a time length between 12 hours and 48 hours, a time length between 16 hours and 40 hours, a time length between 20 hours and 36 hours, or 24 hours. For example, as the breakdown of the 1 cycle, the dark period is set as a time length between 3 hours and 21 hours, the light period is set as a time length between 3 hours and 21 hours, and the total time length of the dark period and the light period may be set as a time length between 20 hours and 26 hours. For example, as the breakdown of the 24-hour cycle, the dark period may be set as a time length between 3 hours and 21 hours, and the light period may be set as a time length between 3 hours and 21 hours, and the total time length of the dark period and the light period may be 24 hours.

As an embodiment, the present invention also provides a method for producing a protein of interest or a peptide of interest, including: determining conditions for cultivation by the method described above; cultivating a plant body expressing a protein of interest or a peptide of interest under the determined conditions for cultivation; and extracting the protein of interest or the peptide of interest from the cultivated plant body.

For example, extracting the protein of interest or the peptide of interest from the cultivated plant body may be extracting the protein of interest or the peptide of interest from the plant body 3 days to 21 days, 5 days to 14 days, or 7 days to 10 days after the infection. The method may further include purifying the extracted protein of interest or peptide of interest. The plant body can be cultivated, and the protein of interest or the peptide of interest can be extracted and purified by well-known methods.

As specific aspects and the like in the method according to the present embodiment, the specific aspects described above and the like can be applied without limitation.

As an embodiment, the present invention also provides a method for determining conditions for cultivating a plant body expressing a protein of interest or a peptide of interest in a 24-hour cycle including a plurality of temperature zones, including:

> a step of determining an optimum leaf temperature based on a capability to express a protein of interest or a peptide of interest and
> a step of setting a leaf temperature and a number of measurements so that the integrated value of leaf temperatures measured at regular intervals after a plant body is infected with a bacterium or a virus having a polynucleotide encoding the protein of interest or the peptide of interest satisfies the following relationship:

$$(T - 1) \times (C_1 + \cdots + C_n) \le (T_1 \times C_1) + \cdots + (T_n \times C_n) \le (T + 1) \times (C_1 + \cdots + C_n)$$

$$n \times \text{measurement interval (hour)} = 24 \text{ (hour)}$$

$$C_1 + \cdots + C_n = 24 \text{ (hour)}$$

> wherein T is the optimum leaf temperature (°C), $T_1$ to $T_n$ are leaf temperatures (°C), n is the number of measurements (time), and n is an integer of 2 or more and 10 or less.

As an embodiment, the present invention also provides a method for determining conditions for cultivating a plant body expressing a protein of interest or a peptide of interest in a fixed time (C (hour)) cycle including a plurality of temperature zones, including:

> a step of determining an optimum leaf temperature based on a capability to express a protein of interest or a peptide of interest and
> a step of setting a leaf temperature and the number of measurements so that the integrated value of leaf temperatures measured at regular intervals after a plant body is infected with a bacterium or a virus having a polynucleotide encoding the protein of interest or the peptide of interest satisfies the following relationship:

$$(T - 1) \times (C_1 + \cdots + C_n) \le (T_1 \times C_1) + \cdots + (T_n \times C_n) \le (T + 1) \times (C_1 + \cdots + C_n)$$

$$n \times \text{measurement interval (hour)} = C \text{ (hour)}$$

$$C_1 + \cdots + C_n = C \text{ (hour)}$$

> wherein T is the optimum leaf temperature (°C), $T_1$ to $T_n$ are leaf temperatures (°C), n is the number of measurements (time), and n is an integer of 2 or more and 10 or less.

The plant body that expresses the protein of interest or the peptide of interest, the infection of the plant body with the bacterium or the virus having the polynucleotide encoding the protein of interest or the peptide of interest, and the like are as described above.

The method according to the present embodiment is a method for determining the conditions for cultivation in a fixed time (C (hour)) cycle including a plurality of temperature zones.

The plurality of temperature zones means 2 or more temperature zones, and for example, the 1 cycle may include 2 to 5 temperature zones, 2 to 4 temperature zones, 2 to 3 temperature zones, or 3 or 2 temperature zones.

As long as the growth of the plant and the production of the protein of interest are possible, the temperature for cultivation is not particularly limited, the temperature for cultivation may be always constant through all the periods, or may be changed according to the growth of the plant, depending on whether it is day time or night time (in the light period or the dark period), or depending on whether it is before or after the infection. For example, the temperature for cultivation may be 10°C to 35°C, 13°C to 28°C, or 15°C to 25°C. If the 1 cycle includes 3 temperature zones ($T_1$ to $T_3$) of the leaf temperatures, for example, $T_1$ may include a temperature zone between 10°C and 35°C, $T_2$ may include a temperature zone between 10°C and 35°C, and $T_3$ may include a temperature zone between 10°C and 35°C.

The relative humidity for cultivation is as described above.

For example, the fixed time (C (hour)) may be a time length between 12 hours and 48 hours, a time length between 16 hours and 40 hours, a time length between 20 hours and 36 hours, or 24 hours.

In the cultivation after the infection, the illuminance for cultivation may be constant, or may be a plurality of different illuminances through the cultivation period. If the illuminance for cultivation includes a plurality of different illuminances, the illuminances for cultivation may be set corresponding to the plurality of temperature zones, or the illuminances for cultivation may be set regardless of a switch in the temperature zone.

For example, the photosynthetic photon flux density for cultivation in the cultivation after the infection may be $0.1\ \mu\text{mol}\cdot\text{m}^{-2}\cdot\text{s}^{-1}$ or more, $0.1\ \mu\text{mol}\cdot\text{m}^{-2}\cdot\text{s}^{-1}$ to $1000\ \mu\text{mol}\cdot\text{m}^{-2}\cdot\text{s}^{-1}$, or $200\ \mu\text{mol}\cdot\text{m}^{-2}\cdot\text{s}^{-1}$ to $500\ \mu\text{mol}\cdot\text{m}^{-2}\cdot\text{s}^{-1}$. If the photosynthetic photon flux density for cultivation includes a plurality of different photosynthetic photon flux densities, for example, the photosynthetic photon flux density for cultivation may be divided into a dark period and a light period as described above. For example, among the plurality of temperature zones that the 1 cycle includes, a higher illuminance may be set for a higher temperature zone.

The leaf temperatures and the number of measurements are set so that the integrated value of leaf temperatures measured at regular intervals after the plant body is infected with the bacterium or the virus having the polynucleotide encoding the protein of interest or the peptide of interest satisfies the following relationship:

$$(T - 1) \times (C_1 + \cdots + C_n) \leq (T_1 \times C_1) + \cdots + (T_n \times C_n) \leq (T + 1) \times (C_1 + \cdots + C_n)$$

$$n \times \text{measurement interval (hour)} = C \text{ (hour)}$$

$$C_1 + \cdots + C_n = C \text{ (hour)}$$

wherein T is the optimum leaf temperature (°C), $T_1$ to $T_n$ are leaf temperatures (°C), n is the number of measurements (time), and n is an integer of 2 or more and 10 or less.

If the plant body that expresses the protein of interest or the peptide of interest is cultivated in the 24-hour cycle including a plurality of temperature zones, the leaf temperatures and the number of measurements are set so that the integrated value of leaf temperatures measured at regular intervals after the plant body is infected with the bacterium or the virus having a polynucleotide encoding the protein of interest or the peptide of interest satisfies the following relationship:

$$(T - 1) \times (C_1 + \cdots + C_n) \leq (T_1 \times C_1) + \cdots + (T_n \times C_n) \leq (T + 1) \times (C_1 + \cdots + C_n)$$

$$n \times \text{measurement interval (hour)} = 24 \text{ (hour)}$$

$$C_1+ \cdots +C_n = 24 \text{ (hour)}$$

wherein T is the optimum leaf temperature (°C), $T_1$ to $T_n$ are leaf temperatures (°C), n is the number of measurements (time), and n is an integer of 2 or more and 10 or less.

The optimum leaf temperature varies depending on the types of the plant body to be used and the protein of interest or the peptide of interest, and the like, for example, the optimum leaf temperature may be a temperature between 18°C and 37°C, a temperature between 20°C and 30°C, a temperature between 22°C and 27°C, a temperature between 22°C and 25°C, a temperature between 23°C and 25°C, or 23°C. If the plant body is a *Nicotiana* plant, for example, the optimum leaf temperature may be a temperature between 22°C and 27°C, a temperature between 22°C and 25°C, a temperature between 23°C and 25°C, or 23°C.

The step of determining the optimum leaf temperature based on the capability to express the protein of interest or the peptide of interest is as described above.

The measurement interval is constant, and may be set, for example, as a time length between 1.2 hours and 24 hours, a time length between from 2 hours and 12 hours, or a time length between 4 hours and 8 hours.

$C_1$ to $C_n$ (hour) indicates times for which the leaf temperature $T_1$ to $T_n$ (°C) are maintained. Since the measurement interval is constant, for example, $C_1$, $C_2$ and $C_3$ are each 8 (hour) in the case where C is 24 (hour), and the number of measurements n is 3.

The integrated value of the leaf temperatures means $(T_1 \times C_1) + \cdots + (T_n \times C_n)$.

The measurement of the leaf temperature is as described above.

The number of measurements n in the formulas described above may be 2 or more, 3 or more, 2 to 10, 2 to 8, 2 to 6, 2 to 5, 2 to 4, or 2 to 3, or may be 2 or 3.

As the specific aspects and the like in the method according to the present embodiment, the specific aspects and the like described above can be applied without limitation.

As an embodiment, the present invention also provides a method for producing a protein of interest or a peptide of interest, including: determining conditions for cultivation by the method described above; cultivating a plant body expressing a protein of interest or a peptide of interest under the determined conditions for cultivation; and extracting the protein of interest or the peptide of interest from the cultivated plant body.

For example, the method for producing the protein of interest or the peptide of interest may further include a step of extracting the protein of interest or the peptide of interest from the plant body 4 to 14 days, 6 to 9 days, or 7 days after the infection. The method may further include a step of purifying the extracted protein of interest or peptide of interest. The step of extracting the protein of interest or the peptide of interest and the step of purifying the protein of interest or the peptide of interest can be performed by well-known methods.

As the specific aspects and the like in the method according to the present embodiment, the specific aspects and the like described above can be applied without limitation.

**Examples**

1. Preparation of plant biomass

(1) Sowing

A seedling raising pot (TS-10.5, Tokai Chemical) was filled with a level potful of culture soil, and 2 to 3 *Nicotiana benthamiana* seeds were sown. Then, a plurality of the seedling raising pots was lined up in seedling raising boxes for medium seedlings (AZ-032, AnzenKougyou Co., Ltd.), bottom surface watering was performed for 30 minutes, and the pots were coated with a covering sheet for agriculture (PAOPAO 90, Mitsubishi Chemical Agri Dream Co., Ltd.). The covering sheet for agriculture was removed on the fifth to seventh day.

(2) Growing

The plants after the sowing were grown at a cooled room temperature of 22°C in a photocycle of a daytime of 16 hours and a nighttime of 8 hours for 14 days. After the grown plants sprouted, and the plants were thinned out so that 1 plant remained per 1 pot. After the thinning out, the seedling raising boxes for medium seedlings were disposed in a cultivation box at 2 weeks of age. At this time, the seedling raising boxes were disposed so that 32 pots were disposed per 1 cultivation shelf. The environmental conditions and the nutrient solution conditions were adjusted as follows.

<Environmental conditions>

- Temperature: 22°C
- Relative humidity: 30 to 80%
- $CO_2$ concentration: 400 to 700 ppm
- Light source: High output of 4950 lm, HF fluorescent lamp, FHF32EX-N-H (TOSHIBA LIGHTING & TECHNOLOGY CORPORATION)

<Nutrient solution conditions>

As a nutrient solution to be fed to the cultivation shelves, a nutrient solution in which a fertilizer A solution (OAT house No. 1 (OAT Agrio Co., Ltd.) 150 g/L) and a fertilizer B solution (OAT house No. 2 (OAT Agrio Co., Ltd.) 100 g/L) were mixed at a mixing ratio of 1:1, under conditions of electrical conductivity (EC): 1.8 mS/cm and pH (6.0) was used.

2. Infiltration of virus

(1) Production of *Agrobacterium* bacterium transformant

The cDNA of the TMV and PVX genomes in which a polynucleotide encoding immunoglobulin G was inserted was introduced into a T-DNA vector, and an *Agrobacterium* bacterium transformed with the obtained T-DNA vector was produced.

(2) Culture of *Agrobacterium* bacterium

Then, each test tube was charged with 5 mL of LB liquid medium on the day before the infiltration, a 20 mg/mL rifampicin solution was added to a final concentration of 50 $\mu$g/mL, and a 50 mg/mL kanamycin solution was added to a final concentration of 25 $\mu$g/mL. Next, 100 $\mu$L of a glycerol stock of the *Agrobacterium* bacterium transformant was added, and shaking culture (230 rpm) was performed at 28°C for around 24 hours to obtain a culture solution.

(3) Preparation of infiltration buffer

The culture solution of the above mentioned (2) was collected and 10 times diluted with phosphate buffered salts (PBS) (solution obtained by diluting 10 $\times$ PBS Liquide Concentre, OmniPur(R) with purified water 10 times and subjecting the dilution to cold storage). The OD value that was the value of the light density determined by measuring the dilution for the absorbance at 660 nm was measured with a simple optical density (OD) monitor (mini photo 518R, TAITEC CORPORATION), and it was confirmed that the OD value was 2.0 or more. Then, 5.3 L of purified water, 130 mL of 0.5 M 2-morpholinoethanesulfonic acid (2-(N-morpholino)ethanesulfonic acid, MES) (pH 5.4), and 65 mL of 1 M $MgSO_4$ were added to a wide-mouthed bucket, and the wide-mouthed bucket was disposed in a vacuum desiccator (300G, AS ONE CORPORATION), and the mixture was stirred. A solution obtained by adjusting the final concentrations of MES and $MgSO_4$ to 10 mM and 10 mM, respectively, the pH to 5.4, and the $OD_{660}$ to 0.002 was used as an infiltration buffer.

(4) Infiltration of virus

Infiltration was performed into the plants that were 5 weeks of age in the operation shown below. The terrestrial parts of the plants were soaked in the infiltration buffer of the above mentioned (3) with the root parts of the plants up. A pole was placed across the mouth of the bucket and used as a stopper so that the culture soil parts were not soaked in the infiltration buffer. The vacuum desiccator was connected to a vacuum pump by a hose, the vacuum pump was operated with the exhaust cock completely opened, and air was aspirated so that the pressure was -0.092 MPaG. The exhaust cock was closed, the vacuum pump was removed from the vacuum desiccator, and the exhaust cook was opened at once. After the return of the pressure, the plants were taken out of the infiltration buffer, and water drops on the surface of the leaves were gently wiped off. The regulation of the LED output of a growth chamber (FC-700Z3, ESPEC MIC CORP.) adjusts the photosynthetic photon flux density in the chamber to 400 $\mu$mol•m$^{-2}$•s$^{-1}$. The plants were disposed in this chamber and subjected to a photoperiod in which a light period of 16 hours and a dark period of 8 hours were repeated alternately. Leaves of the plants were stabbed from the front side thereof with the probe of a temperature logger (Ondotori TR-52i, T&D Corporation), and the probe was contacted with the leaf on the back side thereof to measure the leaf temperature. The room temperature of the growth chamber was set as any leaf temperature, and the plants were cultivated. The plants were cultivated for 7 days after the infection, measurements were then appropriately performed. The relative humidity, the $CO_2$ concentration, and the photosynthetic photon flux density in the cultivation after the infection were the same as the conditions before the infection.

3. Measurement of expression level of immunoglobulin G

(1) Measurement of growth state

(i) Measurement of leaf weight

Leaves harvested from the plants after the infection and stored at -80°C were used as infected leaves. The stems of all the plant were cut by the culture soil surface or near the planting plates, the number of the plants were counted, and the plant weight was measured with a digital scale (total terrestrial part weight (g)). The true leaves of the plants were cut, and the total leaf weight was measured with a digital scale (true leaf weight (g)).

(ii) Measurement of dry matter weight percentage

The weight of a cut sterilization roll was measured with an electronic balance (weight 1). The infected leaves for measuring the dry weight were packed in the sterilization roll the weight of which was measured, and the sterilization roll was sealed. The weight (weight 2) was measured with an electronic balance, and the sterilization roll was then autoclaved at 121°C for 20 minutes. After the autoclaving, the sterilization roll was placed in a dry heat sterilizer (SI601, Yamato Scientific Co., Ltd.) set at 100°C. The sterilization roll was placed in a dry heat sterilizer (TOKYO RIKAKIKAI CO., LTD.) set at 95°C on the next day. The sterilization roll was taken out of the dry heater on the next day and left to stand at room temperature for around 10 minutes, and the weight (weight 3) was then measured with an electron balance. The dry matter weight percentage was calculated from the following formula.

$$\text{Dry matter weight percentage } (\%) = (\text{Weight 3 - Weight 1})/(\text{Weight 2 - Weight 1}) \times 100$$

(2) Quantitative analysis of expression level of immunoglobulin G

(i) Preparation of crude extract solution

A mortar and a pestle were cooled in liquid nitrogen beforehand, the mortar was charged with the infected leaves stored at -80°C, and infected leaves were milled into powder. The weight of a 1.5-mL tube (weight 4) was measured with a scale. A dispensing spoon was cooled in liquid nitrogen, and a suitable amount of the milled leaves were placed in the 1.5-mL tube, and the weight (weight 5) was measured. An extraction buffer (100 mM Tris, 500 mM NaCl, 5 mM EDTA, 40 mM sodium ascorbate) having a weight equivalent to the milled leaf weight (weight 5 - weight 4) was added, and the mixture was left to stand during refrigeration (around 4°C) for 30 minutes and then centrifuged with a desktop centrifuge (TOMY SEIKO CO., LTD., Multi Spin) for 5 minutes. Then, the 1.5-mL tube was charged with 100 μL of supernatant liquid, and the supernatant liquid was stored at -30°C (extract solution).

(ii) Measurement of immunoglobulin G level

An AlphaLISA immunoassay kit was used for the measurement of immunoglobulin G level. The extract solution was 2000 times diluted with a buffer attached to the kit, and measurement was performed according to the instruction book of the kit. The immunoglobulin G level per DL (drying leaves) (mg/g DL) was calculated from the following formula.

Immunoglobulin G level per DL (drying leaves) (mg/g DL) = Protein concentration in extract solution (measured value obtained with AlphaLISA) × Extract solution amount (defined as twice milled leaf weight)/Milled leaf weight (weight 5 - weight 4) × Dry matter weight percentage (%)/100

Cultivation after the infection was performed under the conditions in the following Table 1.

[Table 1]

|  | Test Example 1 | Test Example 2 | Test Example 3 |
|---|---|---|---|
| Leaf temperature | 19 | 23 | 28 |

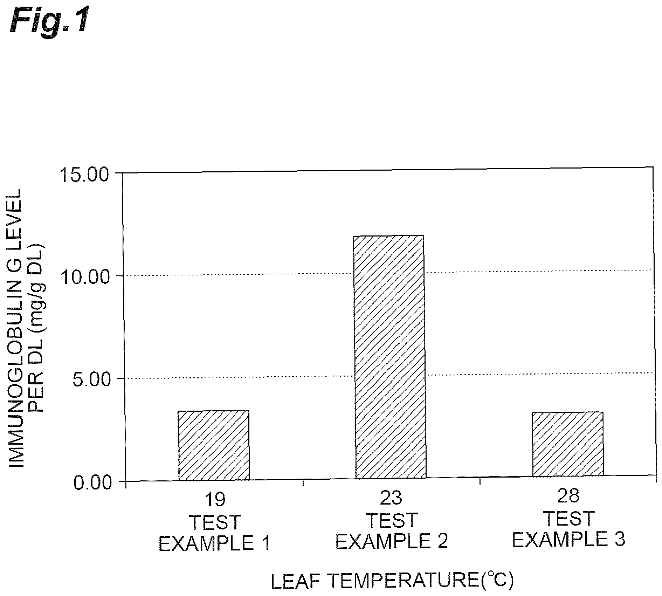

The results of comparing the expression level of immunoglobulin G are shown in Figure 1. It was found from the results of Figure 1 that the immunoglobulin G level per DL (drying leaves) at a leaf temperature of 23°C (Test Example 2) is higher as compared with those at a leaf temperature of 19°C (Test Example 1) and at a leaf temperature of 28°C (Test Example 3). The optimum leaf temperature was set as 23°C from these results. The dark period leaf temperature and the light period leaf temperature were set so as to apply to the following formula from the set optimum temperature, and cultivation was performed under the conditions in Table 2 (Examples 1, 2, and 3).

Dark period leaf temperature (°C) × Dark period time length (hour) + Light period leaf temperature (°C) × Light period time length (hour) = Optimum leaf temperature (°C) × 24 (hour) ± 24

The dark period leaf temperature and the light period leaf temperature were set so as to apply to the following formula from the set optimum temperature under the cultivation condition that the 1 cycle was 12 hours, and cultivation was performed under the conditions in Table 2 (Example 4).

Dark period leaf temperature (°C) × Dark period time length per cycle (hour) + Light period leaf temperature (°C) × Light period time length per cycle (hour) = Optimum leaf temperature (°C) × Total time length of 1 cycle ± 24

[Table 2]

|  | Comparative Example 1 | Example 1 | Example 2 | Example 3 | Example 4 | Comparative Example 2 |
|---|---|---|---|---|---|---|
| Dark period leaf temperature | 27 | 16 | 17 | 26 | 21 | 17 |
| Dark period time length | 8 | 8 | 8 | 8 | 4 | 8 |
| Light period leaf temperature | 27 | 27 | 25 | 22 | 23 | 22 |
| Light period time length | 16 | 16 | 16 | 16 | 8 | 16 |

(continued)

|  | Comparative Example 1 | Example 1 | Example 2 | Example 3 | Example 4 | Comparative Example 2 |
|---|---|---|---|---|---|---|
| Leaf temperature × Time length in dark period (A) | 216 | 128 | 136 | 208 | 84 | 136 |
| Leaf temperature × Time length in light period (B) | 432 | 432 | 400 | 352 | 184 | 352 |
| (A)+(B) | 648 | 560 | 536 | 560 | 268 | 488 |

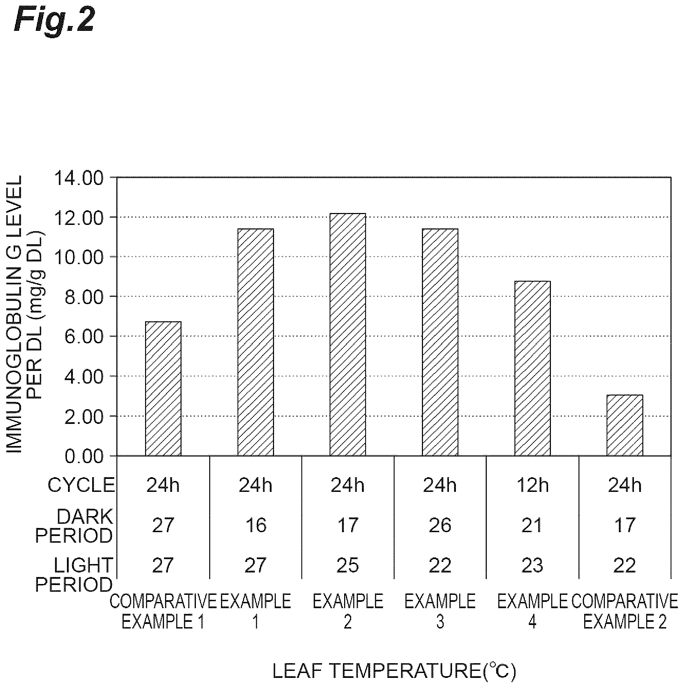

The comparative results of the expression levels of immunoglobulin G are shown in Figure 2. It is found that Examples 1, 2, 3, and 4, applicable to the above formula, exhibit higher immunoglobulin G levels as understood from the immunoglobulin G levels per DL (drying leaves) shown in Figure 2, and Comparative Examples 1 and 2, not applicable to the above formula exhibit lower immunoglobulin G levels. It was shown from these results that the conditions for cultivation under which a high expression level of immunoglobulin G was exhibited were able to be set by the set formula.

**Claims**

1. A method for determining conditions for cultivating a plant body expressing a protein of interest or a peptide of interest in a 24-hour cycle in which a dark period and a light period are repeated alternately, comprising:

   a step of determining an optimum leaf temperature based on a capability to express a protein of interest or a peptide of interest and
   a step of setting light and dark period leaf temperatures and light and dark period time lengths after a plant body is infected with a bacterium or a virus having a polynucleotide encoding the protein of interest or the peptide of interest so as to satisfy the following formula, , the following formula being:

$$\text{Dark period leaf temperature (°C)} \times \text{Dark period time length (hour)} + \text{Light period leaf temperature (°C)} \times \text{Light period time length (hour)} = \text{Optimum leaf temperature (°C)} \times 24 \text{ (hour)} \pm 24.$$

2. The method according to claim 1, wherein the step of determining the optimum leaf temperature comprises determining the optimum leaf temperature based on an expression level of the protein of interest or the peptide of interest.

3. The method according to claim 1 or 2, comprising:
   setting the leaf temperature in the light period higher than the leaf temperature in the dark period.

4. The method according to any one of claims 1 to 3, wherein as a breakdown of the 24-hour cycle, the dark period is set as a time length between 3 hours and 21 hours, the light period is set as a time length between 3 hours and 21 hours, and the total time length of the dark period and the light period is 24 hours.

5. The method according to any one of claims 1 to 4, wherein the optimum leaf temperature is a temperature between 22°C and 27°C.

6. The method according to any one of claims 1 to 5, wherein the dark period leaf temperature is a temperature between 16°C and 27°C, and the light period leaf temperature is a temperature between 16°C and 27°C.

7. The method according to any one of claims 1 to 6, wherein the plant body is a *Nicotiana* plant.

8. The method according to any one of claims 1 to 7, wherein the protein of interest or the peptide of interest is an antibody, an enzyme, a hormone, or an antigen.

9. A method for producing a protein of interest or a peptide of interest, comprising:

   determining conditions for cultivation by the method according to any one of claims 1 to 8;
   cultivating a plant body expressing a protein of interest or a peptide of interest under the determined conditions for cultivation; and
   extracting the protein of interest or the peptide of interest from the cultivated plant body.

10. A method for determining conditions for cultivating a plant body expressing a protein of interest or a peptide of interest in a cycle in which a dark period and a light period are repeated alternately, comprising:

    a step of determining an optimum leaf temperature based on a capability to express a protein of interest or a peptide of interest and
    a step of setting light and dark period leaf temperatures and light and dark period time lengths after a plant body is infected with a bacterium or a virus having a polynucleotide encoding the protein of interest or the peptide of interest so as to satisfy the following formula, the following formula being: Dark period leaf temperature (°C) × Dark period time length per cycle (hour) + Light period leaf temperature (°C) × Light period time length per cycle (hour) = Optimum leaf temperature (°C) × Total time length of 1 cycle ± 24.

11. The method according to claim 10, wherein the step of determining the optimum leaf temperature comprises determining the optimum leaf temperature based on an expression level of the protein of interest or the peptide of interest.

12. The method according to claim 10 or 11, comprising: setting the leaf temperature in the light period higher than the leaf temperature in the dark period.

13. The method according to any one of claims 10 to 12, wherein the dark period is set as a time length between 3 hours and 21 hours, and the light period is set as a time length between 3 hours and 21 hours.

14. The method according to any one of claims 10 to 13, wherein the optimum leaf temperature is a temperature between 22°C and 27°C.

15. The method according to any one of claims 10 to 14, wherein the dark period leaf temperature is a temperature between 16°C and 27°C, and the light period leaf temperature is a temperature between 16°C and 27°C.

16. The method according to any one of claims 10 to 15, wherein the plant body is a *Nicotiana* plant.

17. The method according to any one of claims 10 to 16, wherein the protein of interest or the peptide of interest is an antibody, an enzyme, a hormone, or an antigen.

18. The method according to any one of claims 10 to 17, wherein the 1 cycle is between 20 hours and 36 hours.

19. A method for determining conditions for cultivating a plant body expressing a protein of interest or a peptide of interest in a 24-hour cycle including a plurality of temperature zones, comprising:

    a step of determining an optimum leaf temperature based on a capability to express a protein of interest or a peptide of interest; and
    a step of setting leaf temperatures and a number of measurements so that an integrated value of the leaf temperatures measured at regular intervals after a plant body is infected with a bacterium or a virus having a polynucleotide encoding the protein of interest or the peptide of interest satisfies the following relationship:

$$(T - 1) \times (C_1 + \cdots + C_n) \leq (T_1 \times C_1) + \cdots + (T_n \times C_n) \leq (T + 1) \times (C_1 + \cdots + C_n)$$

$$n \times \text{measurement interval (hour)} = 24 \text{ (hour)}$$

$$C_1 + \cdots + C_n = 24 \text{ (hour)}$$

wherein T is the optimum leaf temperature (°C), $T_1$ to $T_n$ are the leaf temperatures (°C), n is the number of measurements (time), and n is an integer of 2 or more and 10 or less.

20. The method according to claim 19, wherein n is 2 or 3.

21. A method for producing a protein of interest or a peptide of interest, comprising:

determining conditions for cultivation by the method according to claim 19 or 20;
cultivating a plant body expressing a protein of interest or a peptide of interest under the determined conditions for cultivation; and
extracting the protein of interest or the peptide of interest from the cultivated plant body.

22. A method for determining conditions for cultivating a plant body expressing a protein of interest or a peptide of interest in a fixed time (C (hour)) cycle including a plurality of temperature zones, comprising:

a step of determining an optimum leaf temperature based on a capability to express a protein of interest or a peptide of interest and
a step of setting leaf temperatures and a number of measurements so that an integrated value of the leaf temperatures measured at regular intervals after a plant body is infected with a bacterium or a virus having a polynucleotide encoding the protein of interest or the peptide of interest satisfies the following relationship:

$$(T - 1) \times (C_1 + \cdots + C_n) \leq (T_1 \times C_1) + \cdots + (T_n \times C_n) \leq (T + 1) \times (C_1 + \cdots + C_n)$$

$$n \times \text{measurement interval (hour)} = C \text{ (hour)}$$

$$C_1 + \cdots + C_n = C \text{ (hour)}$$

wherein T is the optimum leaf temperature (°C), $T_1$ to $T_n$ are the leaf temperatures (°C), n is the number of measurements (time), and n is an integer of 2 or more and 10 or less.

23. The method according to claim 22, wherein n is 2 or 3.

24. A method for producing a protein of interest or a peptide of interest, comprising:

determining conditions for cultivation by the method according to claim 22 or 23;
cultivating a plant body expressing a protein of interest or a peptide of interest under the determined conditions for cultivation; and
extracting the protein of interest or the peptide of interest from the cultivated plant body.

*Fig.1*

# Fig.2

| | COMPARATIVE EXAMPLE 1 | EXAMPLE 1 | EXAMPLE 2 | EXAMPLE 3 | EXAMPLE 4 | COMPARATIVE EXAMPLE 2 |
|---|---|---|---|---|---|---|
| CYCLE | 24h | 24h | 24h | 24h | 12h | 24h |
| DARK PERIOD | 27 | 16 | 17 | 26 | 21 | 17 |
| LIGHT PERIOD | 27 | 27 | 25 | 22 | 23 | 22 |

LEAF TEMPERATURE(°C)

## INTERNATIONAL SEARCH REPORT

| International application No. |
|---|
| **PCT/JP2022/000548** |

### A. CLASSIFICATION OF SUBJECT MATTER

*A01H 1/00*(2006.01)i; *A01H 3/02*(2006.01)i; *A01G 7/00*(2006.01)i; *A01H 6/82*(2018.01)i
FI:  A01G7/00 601Z; A01H1/00 A; A01H3/02; A01H6/82

According to International Patent Classification (IPC) or to both national classification and IPC

### B. FIELDS SEARCHED

Minimum documentation searched (classification system followed by classification symbols)

A01G7/00, A01H1/00, A01H3/02, A01H6/82, C12N15/00, C12P21/00, C07K14/00, C12M3/00, G01N33/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2016-167990 A (MITSUBISHI CHEM CORP) 23 September 2016 (2016-09-23) claims, paragraphs [0063]-[0064], [0081] | 1-24 |
| A | JP 2014-512802 A (PHILIP MORRIS PRODUCTS S.A) 29 May 2014 (2014-05-29) paragraphs [0237], [0240] | 1-24 |
| A | JP 2010-531136 A (MEDICAGO, INC) 24 September 2010 (2010-09-24) paragraph [0052] | 1-24 |
| A | US 2018/0066275 A1 (BEIJING DABEINONG BIOTECHNOLOGY CO., LTD.) 08 March 2018 (2018-03-08) paragraphs [0115], [0127] | 1-24 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 February 2022** | **15 February 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/000548**

| Patent document cited in search report | | | Publication date (day/month/year) | Patent family member(s) | | | Publication date (day/month/year) |
|---|---|---|---|---|---|---|---|
| JP | 2016-167990 | A | 23 September 2016 | (Family: none) | | | |
| JP | 2014-512802 | A | 29 May 2014 | US paragraphs [0307], [0310] WO | 2014/0296494 2012/098119 | A1 A2 | |
| JP | 2010-531136 | A | 24 September 2010 | US paragraphs [0132]-[0135] WO | 2011/0008837 2008/151440 | A1 A1 | |
| US | 2018/0066275 | A1 | 08 March 2018 | WO EP CN | 2016/127866 3257938 104611307 | A1 A1 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MARILLONNET et al.** In planta engineering of viral RNA replicons: efficient assembly by recombination of DNA modules delivered by Agrobacterium. *Proc Natl Acad Sci US A.,* 26 October 2004, vol. 101 (43), 15546